# EUROPEAN PATENT APPLICATION

(11) **EP 1 782 846 A1**
(43) Date of publication of application: **09.05.2007**
(21) Application number: 05753415.8
(22) Date of filing: 21.06.2005
(51) Int. Cl.: A61L 27/00, A61F 2/14

(54) **CORNEAL EPITHELIAL SHEET AND PROCESS FOR PRODUCING THE SAME**

(30) Priority: 30.06.2004 JP 2004192703
(71) Applicant: Arblast Co., Ltd., Kobe city, Hyogo 650-0047 (JP); Kinoshita, Shigeru, Osaka-shi Osaka 5450035 (JP)
(72) Inventor: KINOSHITA, Shigeru, 5450035 (JP); NAKAMURA, Takahiro; 14-2-611, Yoshidakawaracho,, 6068305 (JP)
(74) Representative: Müller Fottner Steinecke
(86) International application number: PCT/JP2005/011370
(87) International publication number: WO 2006/003818

(57) **Abstract**

A corneal epithelial sheet having a cell layer similar to the anterior epithelium of cornea. A corneal epithelial sheet is obtained through the step (a) of separately preparing a first cell being a self cell and a second cell whose origin is different from that of the first cell, the step (b) of planting the first cell and the second cell on a collagen layer and culturing them, and the step (c)of after proliferation of the cell and second cell resulting in formation of a cell layer, bringing the outermost stratum of the cell layer into contact with the air.

## Description

### Technical Field

The present invention relates to a corneal epithelial sheet and a process for producing the same. The present invention can be used for treating diseases (ocular-surface diseases) that need transplantation of the corneal epithelium. Particularly, the present invention provides an effective means of treating corneal diseases occurring in bilateral eyes.

### Background Art

In surgical treatment for ocular-surface diseases in which the cornea is covered with the conjunctival epithelium to cause haze, at the present time, corneal epithelium transplantation is carried out. However, in refractory keratoconjunctivitis with severe inflammation (Setevens-Johonson syndrome, ocular cicatricial pemphigoid, corneal corrosion, and the like), the prognosis is extremely poor. The biggest reason therefor is thought to be because the corneal epithelium from a different system (allo) having a strong antigenicity is recognized and immunologically rejected by the immune system of a host. In addition, complications caused by systemic and local administration of a large amount of immunosuppressive drugs for preventing the postoperative rejection is one of the largest factors for poor prognosis. On the other hand, when an allogeanic corneal epithelium is used, there is a problem as to the shortage of donors. Therefore, it is thought that transplantation using autologous corneal epithelium tissue is ideal. As to the case of unilateral eye disease (such as corneal corrosion), there have been reports that the corneal epithelium from the normal eye could be successfully transplanted to the affected eye. However, most of the refractory corneal diseases are bilateral-ocular diseases, so that the above-mentioned technique actually cannot be used.
On the other hand, as a surgical treatment method for refractory keratoconjunctive diseases such as Setevens-Johonson syndrome, ocular cicatricial pemphigoid, chemical injury, recurrent pterygium, and the like, which had not been adaptive to cornea transplantation, since 1999, the present inventors have carried out cultured corneal epithelium transplantation using amniotic membrane as a substrate, after having gained the approval of the Ethics Committee of Kyoto Prefectural University of Medicine, and have relatively excellent treatment results (see non-patent document 1 to 3). Furthermore, since 2002, the present inventors have carried out autologous transplantation of cultured oral mucosal epithelium after having gained the approval of the Institutional Review Board for Medical Research involving human subjects of Kyoto Prefectural University of Medicine and have improved the treatment results (see patent document 1 and non-patent document 4).

[patent document 1] Pamphlet of International Publication WO03/043542
[non-patent document 1] Noriko Koizumi et al., Ophthalmology 2001; 108: 1569-74.
[non-patent document 2] Noriko Koizumi et al., Arch Ophthalmol 2001;119:298-300.
[non-patent document 3] Nakamura T et al., Cornea. 22; 70-71: 2003.
[non-patent document 4] Nakamura T et al., Invest Ophthalmol Vis Sci. 2003; 44: 106-16.

### SUMMARY OF THE INVENTION

### [Problems to be Solved by the Invention]

Since most of the refractory keratoconjunctive diseases are bilateral eyes diseases, most of treatments using the cultured corneal epithelium transplantation using amniotic membrane as a substrate must depend upon the use of tissue from another individual (allo). Such a transplantation has problems such as the rejection and bacterial infection, which largely effect on postoperative performances. On the other hand, since treatment method using autologous transplantation of cultured oral mucosal epithelium can use an autologous (auto) oral mucosal epithelium, it is possible to avoid the risk of the rejection, and the like. However, there are problems, for example, a problem of transparency of a cultured epithelial sheet, and a case in which angiogenesis occurs after operation. Further development of the treatment is expected in the future.

### [Means to Solve the Problems]

Under the above-mentioned circumstances, the present inventors have sought for a further progressive probability of reconstructing an ocular-surface using autologous tissue. As a result, the present inventors have reached a new idea of constructing a corneal epithelium-like mucosal epithelial layer by preparing an autologous cell and a cell whose origin is different from that of the autologous cell, and hybridizing both cells. Then, the present inventors chose an oral mucosal epithelial cell as the autologous cell and chose a corneal epithelial cell as the cell whose origin is different from that of the oral mucosal epithelial cell, and made an attempt to construct a corneal epithelial sheet. As a result, it was confirmed that a cell group, in which cells derived from the two kinds of cells had been hybridized, formed a differentiated and layered structure of mucosal epithelial layers. Furthermore, from the transplantation experiment using an animal, it was confirmed that the thus obtained mucosal epithelial layer kept homeostasis of the ocular-surface and had excellent survival.
Herein, a living organism includes various mucosal epithelium tissues. The tissues have many common features peculiar to the mucosal epithelium. When this fact is taken into consideration, for example, conjunctival epithelial cells, nasal mucosal epithelial cells, and the like, although they are different from the cells constituting the other mucosal tissue, can be used as the autologous cells for forming a corneal epithelium-like mucosal epithelial layer as mentioned above similar to the oral mucosal epithelial cells. In particular, since conjunctival epithelial cells are cells constituting neighboring tissue of the corneal epithelium, they are thought to be an effective source of cells.
Furthermore, in general, cells forming a tissue are generated from precursor cells or undifferentiated cells by receiving external signal, and the like. Taken this fact into consideration, as long as they have differentiation potency to the above-mentioned cells (oral mucosal epithelial cells, conjunctival epithelial cells, and the like), the undifferentiated cells can be used as the autologous cells for forming a mucosal epithelial layer, similar to the above-mentioned cells.
On the other hand, as shown in the below-mentioned Examples, in the case where human oral mucosal epithelial cells and human amniotic membrane epithelial cells are hybridized, it was experimentally confirmed that a mucosal epithelial layer similar to the corneal epithelium could be prepared. That is to say, it was determined that a corneal epithelium-like mucosal epithelial layer could be formed even in the case where hybridization is carried out by using cells other than the corneal epithelial cells as the cells to be combined with the oral mucosal epithelial cell that is an autologous cell. Herein, it is assumed that the promising candidate cells to be used similarly to the amniotic membrane epithelial cells include the conjunctival epithelial cells constituting the neighboring tissue of the corneal epithelium.

As mentioned above, the present inventors have made keen examinations and confirmed that a corneal epithelium-like mucosal epithelial layer can be constructed by culturing and hybridizing at least two kinds of cells including autologous cells. The present invention was made based on the above-mentioned results and provides the following configurations.
That is to say, as the first aspect, the present invention provides a corneal epithelial sheet comprising: a cell layer including a first cell that is an autologous cell and a second cell whose origin is different from the first cell, the first cell and the second cell being layered.
In one embodiment of the present invention, the first cell is at least one autologous cell selected from the group consisting of a cell derived from oral mucosal epithelium, a cell derived from conjunctival epithelium, a cell derived from nasal mucosal epithelium, and a cell derived from other mucosal epithelium, as well as a cell derived from an undifferentiated cell capable of constructing any of the mucosal epitheliums; and the second cell is at least one cell selected from the group consisting of a cell derived from corneal epithelium, a cell derived from conjunctival epithelium, and a cell derived from amniotic membrane epithelium.
In another embodiment of the present invention, the first cell is a cell derived from oral mucosal epithelium.
In a further embodiment of the present invention, the second cell is a cell derived from corneal epithelium or a cell derived from amniotic membrane epithelium.
As another aspect, the present invention provides a corneal epithelial sheet further including a collagen layer in addition to the cell layer. In this embodiment, the cell layer is formed on the collagen layer. It is preferable that the collagen layer is derived from amniotic membrane or amniotic membrane from which the epithelium has been removed.
It is preferable that cell layer in the corneal epithelium sheet of the present invention comprises at least one of the following properties or characteristics;
cells of the outermost layer are not cornified;
cells of the outermost layer are flat-shaped; and
a barrier function is provided.

As the second aspect, the present invention provides a process for producing a corneal epithelial sheet. The production process of the present invention includes:
a) separately preparing a first cell being an autologous cell and a second cell whose origin is different from that of the first cell;
b) seeding the first cell and the second cell on a collagen layer and culturing them; and
c) after proliferation of the first cell and second cell resulting in formation of a cell layer, bringing the surface of the cell layer into contact with the air.
In one embodiment of the present invention, the first cell is at least one autologous cell selected from the group consisting of an oral mucosal epithelial cell, a conjunctival epithelial cell, a nasal mucosal epithelial cell, and other mucosal epithelial cell, as well as an undifferentiated cell capable of constructing any of the mucosal epitheliums; and the second cell is at least one cell selected from the group consisting of a corneal epithelial cell, a conjunctival epithelial cell, and an amniotic membrane epithelial cell.
In another embodiment of the present invention, the first cell is an oral mucosal epithelial cell.
In a further embodiment of the present invention, the second cell is a cell of another individual.
In a further embodiment of the present invention, the second cell is a corneal epithelial cell or an amniotic membrane epithelial cell.
The step b is carried out under any of conditions,
1) in coexistence of supporting cells; and
2) in coexistence of supporting cells and in a state in which an isolation membrane with a pore size through which the supporting cell cannot pass exists between the supporting cells and the collagen layer.
   Preferably, the collagen layer is derived from amniotic membrane or amniotic membrane from which the epithelium has been removed.

### BRIEF DESCRIPTION OF THE DRAWINGS

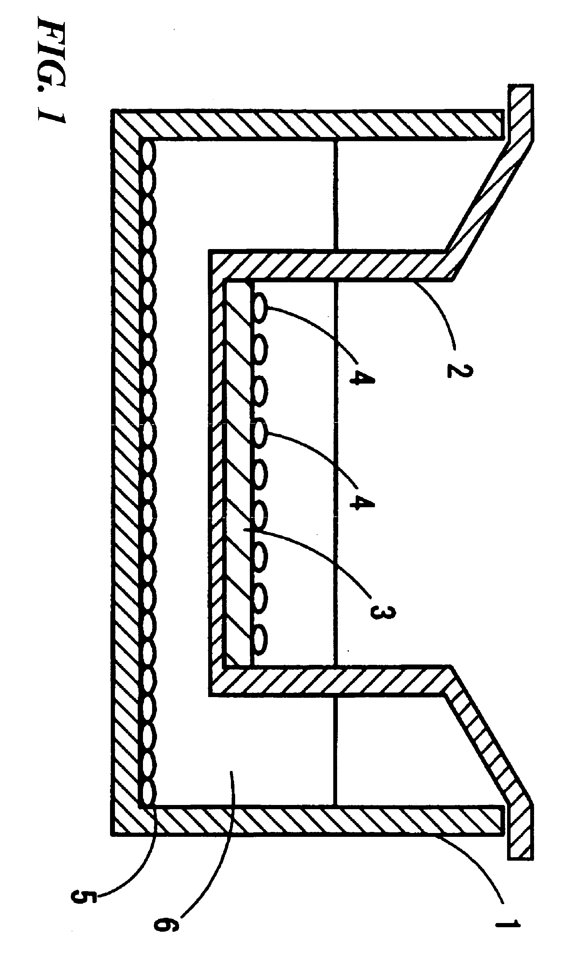

Fig. 1 is a cross-sectional view schematically showing a state of an instrument, etc. when oral mucosal epithelial cells and corneal epithelial cells are cultured on amniotic membrane. In a culture dish 1, a culture insert 2 is disposed. On the bottom surface of the culture dish 1, a 3T3 cell layer 5 is formed. Furthermore, on the bottom surface of the culture insert 2, amniotic membrane 3 is placed, and oral mucosal epithelial cells and corneal epithelial cells 4 are cultured thereon. Reference numeral 6 denotes a culture medium.
   1: culture dish (first container), 2: culture insert (second container), 3: amniotic membrane, 4: oral mucosal epithelial cells and corneal epithelial cells, 5: 3T3 cell layer, 6: culture medium.

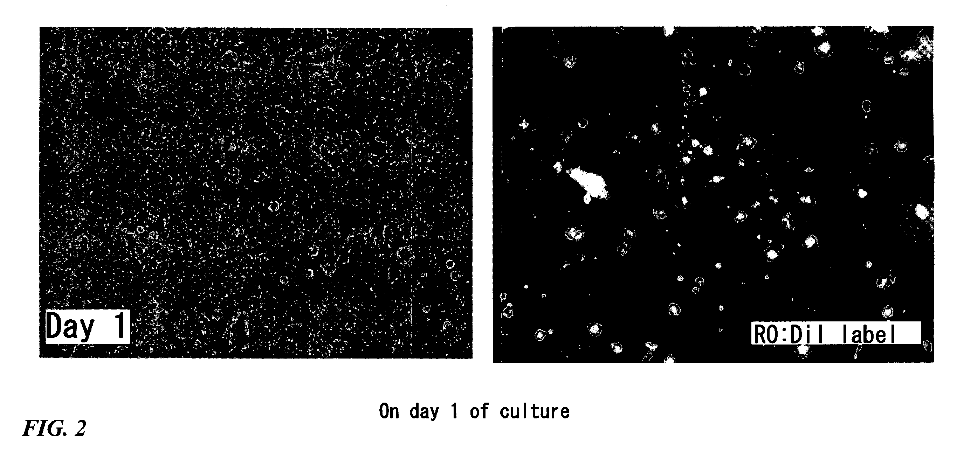

Fig. 2 shows an optical microscope image (left) and a fluorescence stain image (right) on day 1 of culture of oral mucosal epithelial cells and corneal epithelial cells on the amniotic membrane. RO represents a rabbit oral epithelium. In the fluorescence stain image, a Dil signal (orange color) showing the presence of cells derived from the oral mucosal epithelial cell was observed.

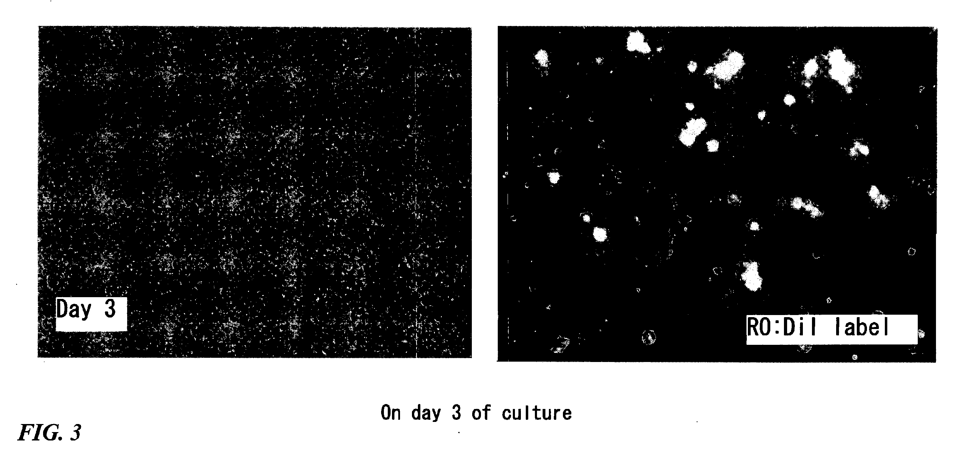

Fig. 3 shows an optical microscope image (left) and a fluorescence stain image (right) on day 3 of culture of the oral mucosal epithelial cells and corneal epithelial cells on the amniotic membrane. RO represents a rabbit oral epithelium. In the fluorescence stain image, a Dil signal (orange color) showing the presence of cells derived from the oral mucosal epithelial cell was observed.

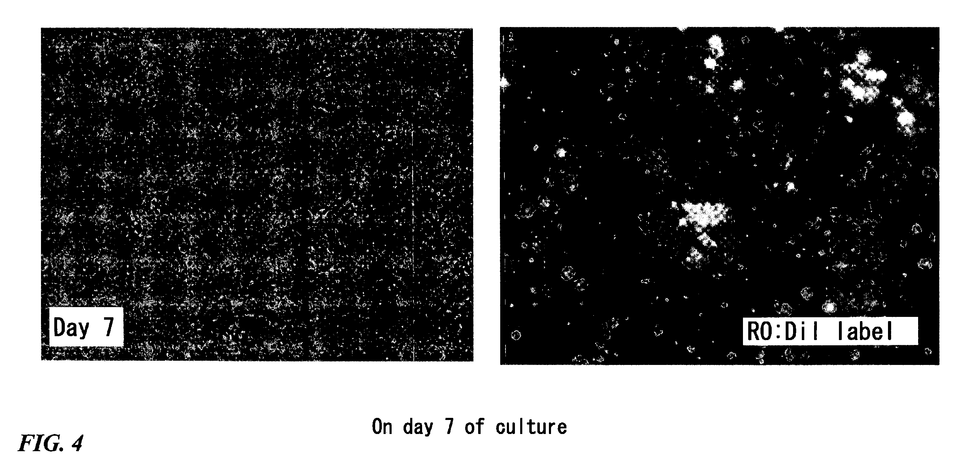

Fig. 4 shows an optical microscope image (left) and a fluorescence stain image (right) on day 7 of culture of the oral mucosal epithelial cells and corneal epithelial cells on the amniotic membrane. RO represents a rabbit oral epithelium. In the fluorescence stain image, a Dil signal (orange color) showing the presence of cells derived from the oral mucosal epithelial cell was observed.

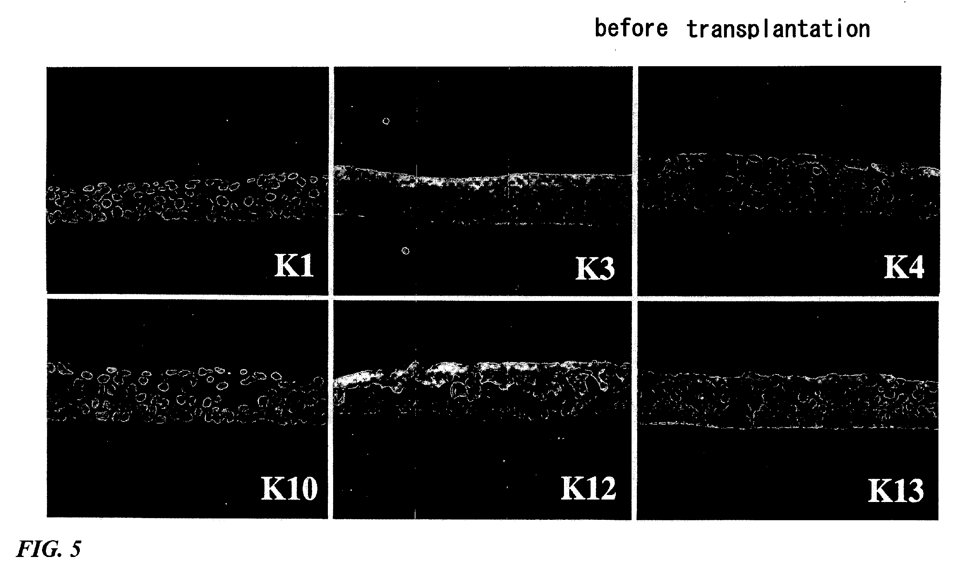

Fig. 5 shows results of immunostaining the cell layer formed on the amniotic membrane. The left images show the staining properties of keratin 1 (K1) and keratin 10 (K10); the middle images show the staining properties of keratin 3 (K3) and keratin 12 (K12); and the right images show the staining properties of keratin 4 (K4) and keratin 13 (K13).

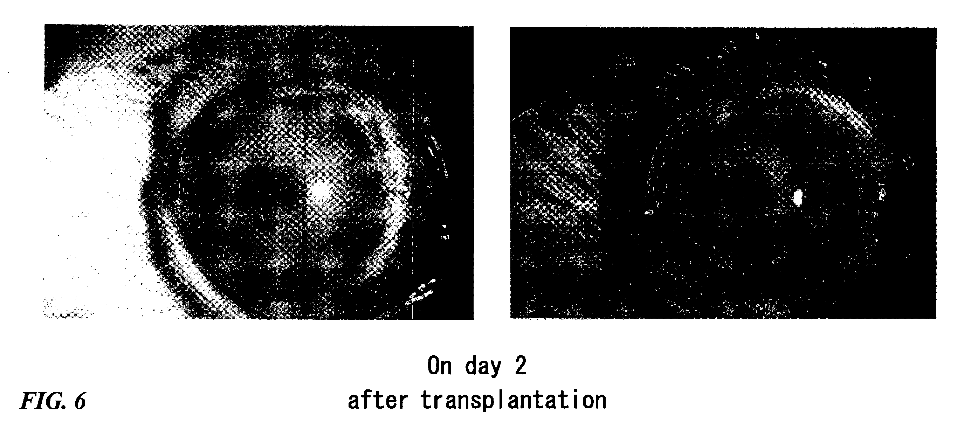

Fig. 6 shows a state of the ocular surface (anterior ocular part) (left image) and a fluorescein stained image of the ocular surface (right image) on day 2 after transplantation of the corneal epithelial sheet according to Example.

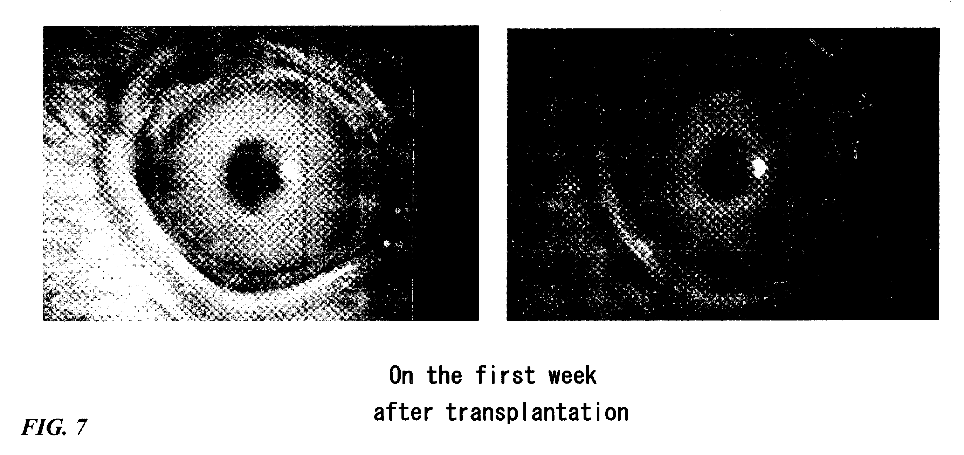

Fig. 7 shows a state of the ocular-surface (anterior ocular part) (left image) and a fluorescein stained image of the ocular-surface (right image) on the first week after transplantation of the corneal epithelial sheet according to Example.

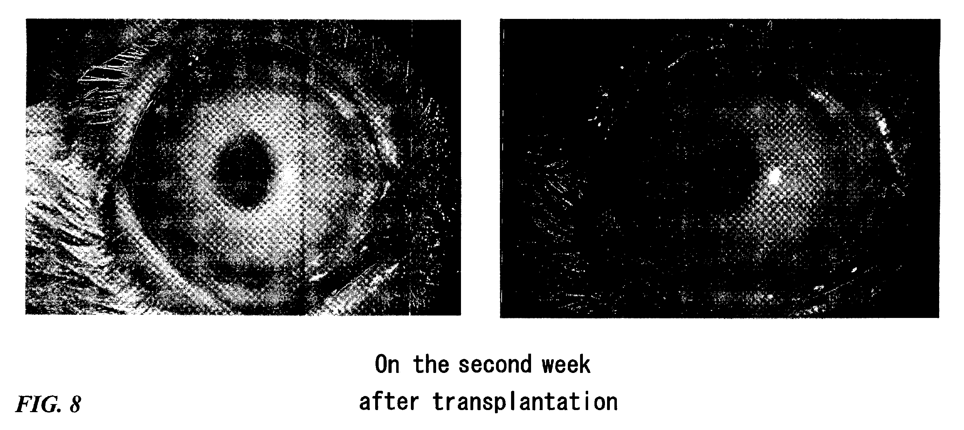

Fig. 8 shows a state of the ocular-surface (anterior ocular part) (left image) and a fluorescein stained image of the ocular-surface (right image) on the second week after transplantation of the corneal epithelial sheet according to Example.

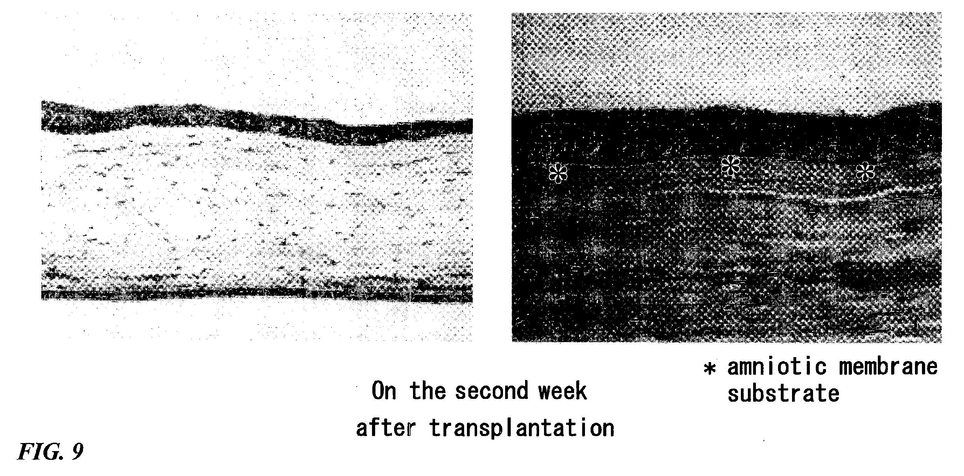

Fig. 9 shows a HE (hematoxylin eosin) stained image of the corneal epithelial sheet on the second week after transplantation.

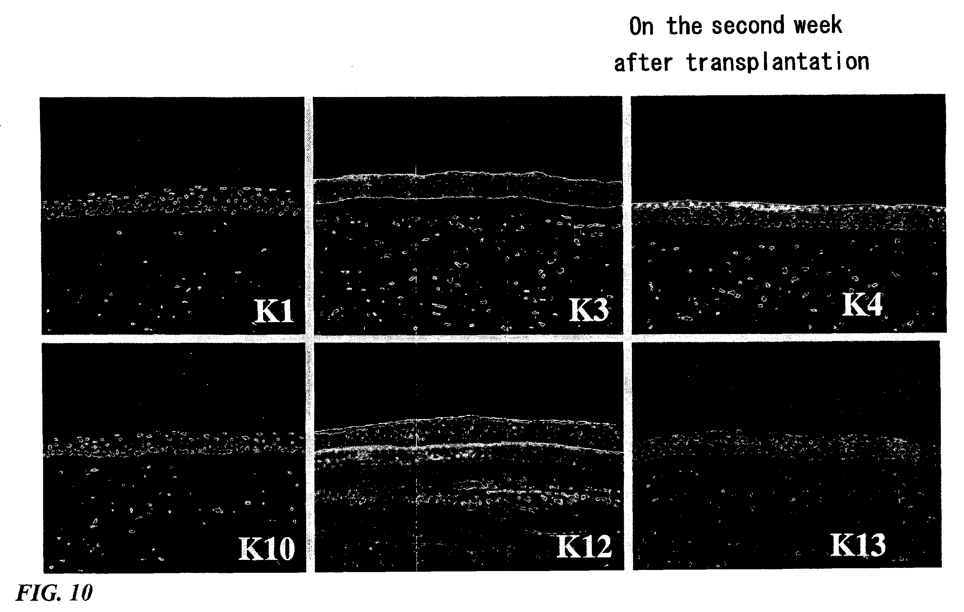

Fig. 10 shows results of immunostaining the corneal epithelial sheet on the second week after transplantation. The left images show the staining properties of keratin 1 (K1) and keratin 10 (K10); the middle images show the staining properties of keratin 3 (K3) and keratin 12 (K12); and the right images show the staining properties of keratin 4 (K4) and keratin 13 (K13).

### BEST MODE OF CARRYING OUT THE INVENTION

The first aspect of the present invention relates to a corneal epithelial sheet. Herein, "corneal epithelial sheet" is a sheet-like structure including a characteristic similar to the corneal epithelium in at least a part of the structure.
The corneal epithelial sheet of the present invention includes a characteristic cell layer. The cell layer includes an autologous cell (herein, also referred to as "a first cell") and a cell whose origin is different from the autologous cell (herein, also referred to as "a second cell"), and a multi-layered structure is formed by these cells. In this specification, the formation from two kinds or more of cells whose origins are different from each other in this way is also referred to as "hybridization." On the other hand, "multi-layered" means being formed of a plurality of cell layers. The corneal epithelial sheet of the present invention includes typically about 4 to 8 layers of cells.
The form (state of hybridization) including cells in the cell layer is not particularly limited. For example, each kind of cells may be dispersed or any kind of cells (or plural kinds of cells) may be present in a group. Alternatively, the content of each kind of cells may not be constant over the cell layer.

The first cell according to the present invention is an autologous cell. The "autologous" herein denotes a subject using the corneal epithelial sheet according to the present invention, that is, a person (recipient) undergoing transplantation. On the other hand, persons other than the "autologous" person denote "another individual."
The kinds of the first cells are not particularly limited as long as they are capable of forming a corneal epithelium-like mucosal epithelial layer when they are hybridized with the below mentioned second cells. An example of the first cell can include a cell derived from oral mucosal epithelium, a cell derived from conjunctival epithelium, a cell derived from nasal mucosal epithelium, or a cell derived from an undifferentiated cell (i.e. a stem cell of mucosal epithelium) capable of constructing any of the mucosal epitheliums. In this specification, "derived from or origin" is used to intend to specify a starting material. Therefore, for example, the cell derived from (or whose origin is) the oral mucosal epithelium is a cell constructed by using an oral mucosal epithelial cell as a starting material. Furthermore, in the present invention, "an undifferentiated cell capable of constructing the mucosal epithelium" denotes a cell having differentiation potency to the mucosal epithelium. For example, an undifferentiated cell capable of constructing the oral mucosal epithelium refers to as a cell capable of being differentiated to an oral mucosal epithelial cell. Specific examples of the undifferentiated cell can include a precursor cell or a stem cell forming a specific tissue such as oral mucosal epithelium, conjunctival epithelium, or the like, or an epithelial stem cell having lower degree of differentiation, and the like.

The cell layer according to the present invention may include two different kinds or more of the first cells. For example, a cell layer may be constructed in a state in which a cell derived from the oral mucosal epithelium and a cell derived from the conjunctival epithelium are contained.

The "oral mucosal epithelium" according to the present invention includes an oral inner marginal mucosa epithelium part, a labial part, a palate part, a buccal part, and the like. Whether such cells are derived from the oral mucosal epithelium can be confirmed by observing the expression of keratin 4 or keratin 13, which are specific to the oral mucosal epithelium as an index. Alternatively, it can be also confirmed by observing that keratin 3 is expressed as an index. This keratin 3 is known to be one of the keratins specific to the cornea but it is confirmed to be expressed also in the oral mucosal epithelium. Note here that it can be said that it is preferable to use oral mucosal epithelial cells as materials for producing compositions for corneal epithelium transplantation from the viewpoint in that this keratin 3 specific to the cornea is expressed.
On the other hand, also by examining the expression of genes specific to an oral mucosal epithelial cell, it can be confirmed that the cells are derived from the oral mucosal epithelium.
Similarly, as to cells derived from a tissue other than the oral mucosal epithelium, the derivation thereof can be confirmed by examining a marker specific to the tissue or gene expression.

A specific example of the second cell can include a cell derived from the corneal epithelium, the conjunctival epithelium or the amniotic membrane epithelium. Among them, it is preferable that the second cell is a cell derived from the corneal epithelium or the conjunctival epithelium. A cell layer constructed by using cells derived from an ocular surface tissue can have a property closer to that of the corneal epithelium. It is particularly preferable that the second cell is a cell derived from the corneal epithelium. It is advantageous because the cell layer having a further closer property to that of the corneal epithelium can be obtained.
The second cell may be an autologous cell or a cell of another individual. When a cell layer is constructed by an autologous cell, a cell layer free from a problem of immunological rejection can be obtained. When a cell layer is constructed by a cell of another individual, it becomes easy to obtain cells as a raw material, so that the cell layer is advantageous from the viewpoint of production. The cell layer of the present invention may include different two kinds or more of second cells. For example, a cell layer may be constructed in a state in which a cell derived from the corneal epithelium and a cell derived from the conjunctival epithelium are included.

Whether a cell layer in the corneal epithelial sheet of the present invention is derived from the corneal epithelium can be confirmed by observing that keratin 3 or keratin 12, which are specific to the corneal epithelium, are expressed as an index. Alternatively, it can be also confirmed by observing that keratin 4 is expressed as an index.
Note here that in the case where cells derived from a tissue other than the corneal epithelium, the derivation thereof can be confirmed by examining a marker specific to the tissue or gene expression.

Preferably, the cell layer in the corneal epithelial sheet of the present invention includes some of the following characteristics or properties. Particularly preferably, the cell layer includes all of the following characteristics or properties.
(1) The cells of the uppermost layer are not cornified. This is one of the features of corneal epithelium. When this feature is observed, the corneal epithelial sheet of the present invention is similar to the corneal epithelium and is expected to exhibit the same function as that of the corneal epithelium. Note here that "cornified" is also referred to as "keratinized", which represents the phenomenon in which keratin is generated in a cell and the cell organelle such as the nucleus is lost. Whether the cells are cornified can be confirmed by observing, for example, the presence or absence of flatness or nucleus in a cell as an index.
(2) The cells of the uppermost layer are flat-shaped. That is to say, an oral mucosal epithelial cell layer is configured by forming a layer of cells having flat shape on a layer of cells having approximately cuboidal shape. It is thought that when the uppermost layer is covered with flat-shaped cells, the tightness between cells is increased and a below-mentioned barrier function is attained. Also in the corneal epithelium, cells in the uppermost layer are flat-shaped. When this feature is observed, the corneal epithelial sheet of the present invention is similar to corneal epithelium and is expected to exhibit the same function as that of corneal epithelium.
(3) A barrier function is provided. The barrier function means a function of preventing liquid, gas, or the like, from infiltrating from the surface or a function of preventing liquid from releasing through the surface layer. When such barrier function is provided, it is possible to maintain moisture (tear) on the surface after transplantation and to prevent more than necessary moisture from being released. The cornea can maintain moisture on the surface thereof as it has a barrier function, and thereby it resists blinking. Therefore, the barrier function is one of the most important features required for a material for cornea transplantation. When this feature is observed, the corneal epithelial sheet of the present invention is similar to the corneal epithelium and is expected to exhibit the same function as that of the corneal epithelium. Whether or not this barrier function is provided can be examined based on the extent of infiltration of solution including an indicator such as Horseradish peroxidase.
   The corneal epithelial sheet of the present invention can be used as transplantation material (substitute for the corneal epithelium) to a patient with damaged cornea or failure cornea, etc. In transplantation, it is preferable that a graft is fixed to and allowed to survive by fixing it to the surrounding tissue with a surgical suture. Furthermore, it is preferable that after transplantation, the surface of the transplanted part is protected by temporarily being covered with a therapeutic contact lens.

In the corneal epithelial sheet in accordance with one embodiment of the present invention, the cell layer is formed on a collagen layer. That is to say, in this embodiment, a collage layer is provided in addition to the cell layer. The collage layer herein is preferably derived from the amniotic membrane. It is advantageous because it is possible to obtain a corneal epithelial sheet with excellent biocompatibility and low immunogenicity due to its high biocompatibility and low immunogenicity of the amniotic membrane. It is preferable to use a collagen layer derived from the amniotic membrane from the viewpoint of production of a corneal epithelial sheet. That is to say, as mentioned below, a corneal epithelial sheet having a collagen layer can be obtained by seeding predetermined cells on the collagen layer as a substrate and culturing them, and the amniotic membrane has a property in which cells are attached and proliferate thereon. Therefore, the use of the collagen layer derived from the amniotic membrane enables excellent adhesion and proliferation of cells and the formation of the cell layer.
It is further preferable that the collage layer is derived from the amniotic membrane from which the epithelium has been removed by, for example, a scraping procedure. It is advantageous because no epithelial components are contained, a corneal epithelial sheet with further reduced immunogenicity can be obtained. Since cells can be adhered and proliferated on the amniotic membrane from which the epithelium has been removed, it is advantageous in terms of production that a high quality corneal epithelial sheet can be constructed for shorter time.
Whether the collagen layer is made of the amniotic membrane from which the epithelium has been removed can be confirmed by examining that a cell of the amniotic membrane epithelial layer is not contained in the collagen layer. Note here that it is preferable that the human amniotic membrane is used as the amniotic membrane.

This corneal epithelial sheet of the present invention can be used as a transplantation material (substitute for the corneal epithelium) for patients with injured or defective cornea, etc. In the case where the corneal epithelial sheet including a collagen layer is used, it is transplanted to the corneal epithelium defective part so that the collagen layer is located to the side of the eyeball. On the other hand, in the case of using a corneal epithelial sheet obtained by forming a cell layer on the collagen layer and then removing the collagen layer, it is transplanted to the corneal epithelium defective part so that the side in which the collagen layer has been present is located to the side of the eyeball.
In transplantation, it is preferable to promote survival of the graft by fixing it to the surrounding tissue with a surgical suture. Furthermore, after transplantation, it is preferable that the surface of the transplanted part is protected by temporarily being covered with a therapeutic contact lens.

The corneal epithelial sheet of the present invention can be prepared by the following process (second aspect of the present invention). The second aspect of the present invention relates to a process for production a corneal epithelial sheet and the following steps.
a) separately preparing a first cell being an autologous cell and a second cell whose origin is different from that of the first cell;
b) seeding the first cell and the second cell on a collagen layer and culturing them; and
c) after proliferation of the first cell and second cell resulting in formation of a cell layer, bringing the surface of the cell layer into contact with the air.

The production process of the present invention is characterized in that two kinds or more of cells are co-cultured. As shown in the below-mentioned Examples (co-culturing the oral mucosal epithelial cells and the corneal epithelial cells), according to the process having such a characteristic, excellent cell proliferation and rapid formation of cell layer according to this were observed. By co-culturing two kinds or more of cells in this way, a cell layer can be formed for a shorter time.

An example of the first cell preferably includes an oral mucosal epithelial cell, a conjunctival epithelial cell, a nasal mucosal epithelial cell or other mucosal epithelial cells, or an undifferentiated cell capable of constructing any of the mucosal epithelium. On the other hand, as the second cell, a corneal epithelial cell, a conjunctival epithelial cell, or an amniotic membrane epithelial cell is preferably used. These cells are harvested from a living tissue in which these cells are present. Specifically, for example, after a part of the tissue where a target cell exists by using a surgical knife, and the like, followed by procedures such as removing the connective tissue and separating cells, and the like. Then, cells are prepared in a shape of a cell suspension (suspension). Note here that the first cell may include two different kinds of cells. Similarly, the second cell may include two different kinds of cells.

It is suggested that oral mucosal epithelium as a preferable harvesting source of the first cell has a stem cell and it is thought to easily induce differentiation of them to cells forming an epithelial cell layer. Furthermore, the use of the oral mucosal epithelial cells has the following advantages: they can be harvested easily; a large number of cells can be harvested; and when a patient with bilateral-eye disease is treated, transplantation material derived from the autologous cells can be prepared. In particular, with the advantage that a patient from which corneal epithelial cells cannot be harvested, transplantation materials derived from autologous cells can be used, it is expected that the clinically important problem about immunological rejection can be significantly solved.
As the oral mucosal epithelial cell, a cell existing in the dental root part (a cell of the oral inner marginal mucosa epithelium), a cell of labial part, a cell of palate part, a cell of buccal part, and the like, can be used. Among them, it is particularly preferable to use a cell of oral inner marginal mucosal epithelial cell because it has a high proliferation ability and low antigenicity. The oral mucosal epithelial cells can be harvested by ablating a site where a targeted cell exists by using a scalpel or by scraping it out. Oral inner marginal mucosal epithelial cell can be harvested from the oral inner marginal mucosal epithelial cell that was separated from enamel cement transition portion. Note here that in order to remove impurities such as connective tissue, preferably a treatment with enzyme such as Dispase or trypsin, etc., filtration treatment are carried out.
Oral mucosal epithelial cells harvested from an oral cavity of an individual other than a patient to whom a corneal epithelial sheet constructed according to the present invention is to be transplanted may be used. However, when taking the immunological rejection into consideration, preferably the oral mucosal epithelial cell from a patient him/herself is harvested and cultured.
Since mucous membrane of oral cavity has high proliferation ability and the wound is generally healed by oral administration of antibiotic, disinfection with, for example, Isodine, for several days after operation. Therefore, it is thought that invasion to a patient himself/herself due to the harvest of mucosa.

On the other hand, as the second cell, another individual's (allo) corneal epithelial cell can be preferably used. As such a corneal epithelial cell, donor's eyeball free from infection is available from, for example, eye bank (Northwest eye bank, etc.). The cells that can be used as the second cell are not limited to the corneal epithelial cell. The conjunctival epithelial cell, an amniotic membrane epithelial cell, and the like, may be used. However, when the corneal epithelial cells constituting the corneal epithelium in a living organism or the conjunctival epithelial cells existing in the vicinity thereof are employed, a corneal epithelial sheet capable of reproducing the property of the corneal epithelium more excellently. As shown in the below-mentioned Examples, when the corneal epithelial cell is used as the second cell, it was confirmed that a cell layer similar to the corneal epithelium could be constructed. This fact supports the above-mentioned prediction and supports that the corneal epithelial cell is particularly preferable for the second cell. On the other hand, as mentioned in the below-mentioned Example, it was confirmed that when the amniotic membrane epithelial cell was used as the second cell, a cell layer capable of excellently reproducing the properties required for the cornea could be formed. This fact shows that the amniotic membrane epithelial cells can be preferably used as the second cell.

Autologous cells can be used as the second cell. However, when another individual's cells are used, the cells can be obtained more easily. For example, when a corneal epithelial sheet for the treatment of a patient with bilateral eye disease is produced, the corneal epithelial cells as the second cell are available.

The respectively prepared first cell and the second cell (hereinafter, also referred to as "first cell, and the like") are seeded on a collagen layer and cultured (step b). In general, the first cell and the second cell, which are prepared in a form of a cell suspension, are dripped on a collage layer and cultured.
Typically, the seeding of the first cells and the seeding of the second cells are carried out simultaneously (herein, "simultaneously" includes not only a case where the seedings are carried out literally simultaneously but also a case where the first seeding is carried out and then the second seeding is carried out without substantial time interval). However, the first and second cells may be seeded at different timing. For example, the second cells may be seeded several minutes to several tens of minutes after the first cells are seeded. Thus, by stagerring the time of seeding cells, for example, a cell layer in which a region rich in the cells derived from the first cell is localized can be constructed. Thereby, a structure of the cell layer and the property thereof can be changed or adjusted.

The ratio of the first cells and the second cells to be seeded is not particularly limited. Typically, the number of the first cells to be seeded is substantially the same as that of the second cells to be seeded. In an experiment in which the oral mucosal epithelial cells were used as the first cells and the corneal epithelial cells were used as the second cells, the ratios of the number of the first cells: second cells were changed to 3 : 7, 5 : 5, and 7 : 3 and comparison was made. As a result, no difference in terms of the cell proliferation and layering were clearly observed among them (data are not shown).

Herein, the kinds of collagens as a material of the collagen layer are not particularly limited, and type I collagen, type III collagen, and type IV collagen, and the like, can be used. A plural kinds of collagens can be used in combination thereof. Such collagens can be extracted and purified from the connective tissue of the skin and cartilage, etc. of animals such as swine, bovine, sheep, etc., by an acid solubilization method, alkali solubilization method, oxygen solubilization method, and the like. Note here that for the purpose of deteriorating the antigenicity, it is preferable that a so-called atherocollagen obtained by removing telopeptide by a treatment with the use of catabolic enzyme such as pepsin, trypsin, etc.
As the collagen layer, it is preferable to use a collagen derived from amniotic membrane, particularly derived from human amniotic membrane. Herein, the collagen layer is "derived from amniotic membrane" broadly means that the collagen gel is obtained by using amniotic membrane as a starting material. Human amniotic membrane is a membrane covering the outermost layer of the uterus and the placenta, and a basal membrane and an epithelium layer are formed on parenchymal tissue that is rich in collagen. Human amniotic membrane can be harvested by, for example, human embryonic membrane, placenta, etc. obtained at the time of afterbirth at delivery. Specifically, the human amniotic membrane can be prepared by treating and purifying the integrated material including human embryonic membrane, placenta, and umbilical cord obtained right after delivery. The method of treating and purifying can employ a method described in, for example, Japanese Patent Unexamined Publication No. 5-5689. That is to say, amniotic membrane is detached from the embryonic membrane obtained at delivery and remaining tissue is removed by a physical treatment such as ultrasonic cleansing and an enzyme treatment, and the like. Then, appropriate cleaning process is carried out and thus the human amniotic membrane can be prepared.
The thus prepared human amniotic membrane can be cryopreserved before use. The human amniotic membrane can be frozen in a liquid mixing equal volume ratio of DMEM (Dulbecco's modified Eagle's medium) and glycerol at, for example, -80°C. By the cryopreservation, not only the improvement in operation but also reduction of the antigenicity can be expected.
Intact amniotic membrane may be used as a collagen layer but it is preferable that amniotic membrane from which the epithelium is removed by a scraping treatment, etc. is used. For example, after thawing, cryopreserved human amniotic membrane is subjected to a treatment with EDTA or proteolytic enzyme so as to loosen the adhesion between cells and then the epithelium is scraped by using a cell scraper, etc. Thus, the human amniotic membrane from which the epithelium has been removed can be prepared.

When the human amniotic membrane from which the epithelium has been removed is used as the collagen layer, the first cells, and the like, are preferably seeded on the side of the collagen layer with the side where the epithelium has been removed and exposed (i.e., the side of the basal membrane). It is advantageous because it can be thought that this face side is rich in type IV collagens and the seeded first cells, and the like, can be proliferated and layered well.
The first cells and second cells can be seeded on the collagen layer so that, for example, the cell density becomes about 1×10³ cells/cm² or more, preferably in the range from about 1×10³ cells/cm² to about 1×10⁵ cells/cm², and further preferably in the range from about 1×10⁴ cells/cm² to about 1×10⁵ cells/cm².

It is preferable that the first cells and the like are cultured in the presence of supporting cells. The supporting cell is also referred to as a feeder cell and supplies a culture medium with a growth factor, etc. When the first cells and the like are cultured in the coexistence of the supporting cells, the proliferation efficiency of the cells is improved. As the supporting cells, for example, a 3T3 cell (Swiss mouse 3T3 cell, mouse NIH3T3 cell, 3T3J2 cell, etc.) and the like may be used. Among them, it is preferable to use a mouse NIH3T3 cell as a supporting cell from the viewpoint of proliferation efficiency, ease in handling, etc.
It is preferable that the supporting cells are inactivated by using mitomycin C, etc. It is advantageous because the inhibition of the proliferation of the first cells and the like due to the proliferation of the supporting cells themselves is prevented, and the proliferation efficiency of the first cells and the like is enhanced. Such inactivation can be carried out by a radiation treatment, and the like.

The cell density of the supporting cells may be, for example, about 1×10² cells/cm² or more, preferably in the range from about 1×10² cells/cm² to about 1×10⁷ cells/cm², and further preferably in the range from about 1×10³ cells/cm² to about 1×10⁵ cells/cm². As to the ratio with respect to the number of the first cells and the second cells, culture may be carried out under the conditions in which the supporting cells to be used may be, for example, 1/10³ times to 1×10² times, and preferably 1/10² times to 1 time as the total number of the first cells and the second cells. When the number of the supporting cells is small, the proliferation rate of the first cells and the second cells is lowered; and when it is too small, excellent layered structure of the first cells and the like is cannot be obtained. On the other hand, it is not preferable that the number of the supporting cells is too large, because the proliferation rate of the oral mucosal epithelial cells is lowered.

When the first cells are cultured in the coexistence of supporting cells, it is preferable that an isolation membrane having a pore size through which the supporting cells cannot path is provided between the supporting cells and the collagen layer. The use of the isolation membrane makes it possible to prevent the supporting cells from entering the side of the collagen layer (i.e. the side of oral mucosal epithelial cells) at the time of culturing. As a result, the supporting cells may not be mixed in the finally obtained corneal epithelium-like sheet. This means that a corneal epithelial sheet being free from problem of immunological rejection by the supporting cells can be constructed. This is clinically significant so much.
As the isolation membrane, an isolation membrane having a pore size through which the supporting cells cannot path can be used by appropriately selecting the known membrane. For example, a membrane having a pore size of about 0.4 µm to 3.0 µm made of polycarbonate can be used. A material of the isolation membrane is not particularly limited. In addition to polycarbonate, polyester and the like may be used. Such isolation membranes are on the market and easily available.
An example of the culture method using an isolation membrane may include the following method. Firstly, inactivated supporting cells are seeded and cultured on a container such as a dish (a first container), thereby forming a layer of supporting cells on the surface of the container. Next, a second container, which has a bottom face made of an isolation membrane, is set in the first container so that the bottom face of the second container is located in a culture medium. Then, the collagen layer is formed on the bottom face, that is, on the isolation membrane, a collagen layer is formed. Then, on the collagen layer, the first cells and the like are seeded and cultured.
On bottom surface of the second container, a collagen layer may be previously formed (for example, on the bottom surface of the second container, the amniotic membrane from which an epithelium has been removed is placed. In this state, drying process may be carried out). This second container may be set in the first container in which supporting cells are seeded, and then on the collagen layer, the first cells and the like may be seeded and cultured.

The culture medium used for culturing the first cells and the like is not particularly limited as long as the cells can be proliferated and a layered structure of the cells can be formed. For example, it is possible to use a medium, in which DMEM (Dulbecco's modified Eagle's medium) that is generally used for growing epithelial cells and Ham's F12 medium are mixed with each other at the predetermined ratio, and FBS, growth factor, antibiotics, and the like are added. Specific examples include a mixing medium of DMEM and Ham's F12 medium (mixing volume ratio of 1 : 1) to which FBS (10%), insulin (5 mg/ml), cholera toxin (0.1 nM), epithelial cell growth factor (EGF) (10 ng/ml) and penicillin-streptomycin (50 IU/ml) are added. Furthermore, a mixing medium of DMEM and Ham's F12 medium to which triiodothyronine (e.g. 2 nM), glutamine (e.g. 4 mM), transferrin (e.g. 5 mg/ml), adenine (e.g. 0.18 mM), and/or hydrocortisone (e.g., 0.4 mg/ml) are further added, may be used.

When the first and second cells are cultured on a collagen layer, these cells are proliferated and a cell layer is formed (in this process, at least a part of the cells are thought to be differentiated). After the formation of a cell layer, a step (step (c)) of bringing the surface layer of the cell layer into contact with the air is carried out. Note here that this step herein also is referred to as Air lifting. This step (c) is carried out for differentiation of cells forming the cell layer and inducing the barrier function.
This step can be carried out by lowering the surface of the culture medium by temporarily removing a part of the culture medium by using a dropper, a pipette, and the like, thereby temporarily exposing the surface of the oral mucosal epithelial cell layer to the outside of the culture medium. Alternatively, this step can be carried out by lifting up the oral mucosal epithelial cell layer together with the collagen layer, thereby temporarily exposing the surface from the culture medium surface. Furthermore, by using the tube etc., the air may be fed into the culture medium so as to bring the surface of the cell layer into contact with the air. From the viewpoint of the ease in operation, it is preferable that by lowering the surface of the culture medium, thereby exposing the surface of the cell layer to the outside.
The period when this step (c), that is, the period of time when the uppermost layer of the layered structure of cells is brought into contact with the air differs depending upon the state of the cells, culture conditions, and the like, but the period may be, for example, three days to two weeks, preferably within a week, and further preferably within three days.
According to the above-mentioned method of the present invention, on the collagen layer, a corneal epithelium-like cell layer, in which the first cells and the like are layered, is formed. The thus obtained corneal epithelial sheet together with the collagen layer used as a substrate of the first cell and the like can be used as a transplantation material (substitute for the corneal epithelium) for patients with injured or defective cornea. In this case, the sheet is transplanted to the corneal epithelium defective part so that the collagen layer is located to the side of the eyeball. In transplantation, it is preferable to promote survival of the graft by fixing it to the surrounding tissue with a surgical suture. Furthermore, it is preferable that after transplantation, the surface of the transplanted part is protected by temporarily being covered with a therapeutic contact lens.
Note here that a graft from which a part or all of the collagen layer has been removed may be used. The collagen layer can be removed by appropriately combining a chemical treatment with EDTA, etc., an enzymatic treatment by proteolytic enzyme, etc., and a physical treatment such as scraping by using forceps.

Hereinafter, one example of specific transplantations is described. Firstly, cicatrical tissue is incised in the corneal limbus of a patient with keratoconjunctive. Then, the cicatrices conjunctiva tissue invading into the cornea is ablated so as to expose the parenchyma of cornea, followed by suturing corneal epithelial sheet at a portion slightly inside the limbus. The operative procedure is in principle the same as the operative technique which the institution the present inventors belong have carried out as clinical applications to 70 cases or more (operation procedure relating to a corneal epithelial sheet obtained by culturing a corneal epithelial cell or a corneal epithelial sheet obtained by culturing an oral mucosal epithelial cell). It is thought that the operative procedure can be carried out extremely stably.
Hereinafter, Examples (including experimental examples) of the present invention will be described.

### [Example 1]

### <Production and evaluation of hybridized corneal epithelial sheet>

### 1-1. Harvest of amniotic membrane

After giving a pregnant woman who does not have a systemic complication and would undergo Caesarean section sufficient informed consent together with an obstetrician in advance, the amniotic membrane was obtained during the Caesarean section in the operation room. The operation was carried out cleanly. In accordance with the operation work, the operators washed hands, and then wore a special gown. Before delivery, a clean vat for obtaining the amniotic membrane and physiologic saline for washing were prepared. After delivery, the placenta tissue was transferred to the vat and the amniotic membrane tissue was manually removed from the placenta. A portion where the amniotic membrane and the placenta were strongly adhered to each other was separated with scissors.

### 1-2. Treatment of amniotic membrane

Treatment process of amniotic membrane included: (1) washing, (2) trimming, and (3) storing sequentially in this order. Throughout all the processes, operation is desired to be carried out in a clean draft. For all containers and instruments for use, those sterilized were used, and for dishes, etc. sterilized disposable ones were used. The obtained amniotic membrane was washed for removing blood component attached thereto and further washed in a sufficient amount of physiological saline (0.005% ofloxacin was added). Then, the amniotic membrane was transferred to a phosphate buffer solution (PBS) in a dish and cut and divided into the size of about 4 × 3 cm with scissors. The divided pieces of amniotic membrane were stored in several dishes filled with a stock solution, and thereafter amniotic membranes in good condition were selected among them.

### 1-3. Storage of amniotic membrane

One cc each of stock solution was placed in 2 cc sterilized cryotube and one sheet each of the amniotic membrane, which had been obtained, washed and selected, was placed and labeled, then stored in a refrigerator at -80°C. For the stock solution, 50% sterilized glycerol in DMEM (Dulbecco's Modified Eagle Medium: GIBCOBRL) was used. The expiration date for use of stored the amniotic membrane was determined at three months and expired amniotic membrane was disposed of by incineration.

### 1-4. Treatment of amniotic epithelium

The amniotic membrane was subjected to treatment for removing the epithelium and then used for culture. First of all, the amniotic membrane stored at -80°C was thawed at room temperature, and then well washed in sterilized a phosphate buffer solution (PBS) in the dish. After washing, the amniotic membrane was stored in a 0.02% EDTA solution (Nacalai tesque) at 37°C for 2 hours, and then the epithelium was mechanically scraped off by using a cell scraper (Nunc, USA) and used as a substrate for culture. Note here that, it was confirmed that one layer of the amniotic epithelium was completely scraped by this procedure process by the optical microscope and electron microscope (scanning electron microscope) operations.

### 1-5. Harvest of oral mucosal epithelial cells

In 6-week old Japanese white rabbit, tooth was pulled out. Then, the oral mucosal epithelium was carefully separated from the enamel cement transition portion. Note here that a series of operations were carried out by using sterilized instruments as antiseptically as possible.
The obtained oral mucosal epithelium was immersed twice in a phosphate buffer solution (PBS) containing 50 IU/ml penicillin streptomycin and Gentacin for 30 minutes under the condition of room temperature. Thereafter, the tissue was immersed in a phosphate buffer solution (PBS) containing 1.2U Dispase (Nacalai tesque) for one hour at 37°C and immersed and treated in 0.05% trypsin-EDTA solution (GBCOBRL) for 30 minutes so as to separate cells. An enzyme activity was stopped by immersing in DMEM containing 10% fetal bovine serum (FBS). Thereafter, excess tissues were removed by using a 60 µm cell-filter so as to isolate the oral mucosal epithelial cell (oral inner margin epithelial cell) (oral mucosal epithelial cell suspension).

### 1-6. Harvest of corneal epithelial cells

In 6-week old Japanese white rabbit (a different rabbit from the rabbit from which the oral mucosal epithelial cell had been harvested), the limbus strip with the size of 5 mm × 10 mm was harvested from the corneal limbus by using a surgical knife.
The obtained tissue strip was immersed twice in a phosphate buffer solution (PBS) containing 50 IU/ml penicillin streptomycin and Gentacin for 30 minutes under the condition of room temperature. Thereafter, the tissue was immersed in a phosphate buffer solution (PBS) containing 1.2U Dispase (Nacalai tesque) for one hour at 37°C and immersed and treated in 0.05% trypsin-EDTA solution (GBCOBRL) for 15 minutes so as to separate cells. An enzyme activity was stopped by immersing in DMEM containing 10% fetal bovine serum (FBS). Thereafter, excess tissues were removed by using a 60 µm cell-filter so as to isolate the corneal epithelial cell (corneal epithelial cell).

### 1-7. Preparation of co-cultured cell

As the co-culture cells (support cells), NIH-3T3 cells (hereinafter, referred to as "3T3 cell") were used. The 3T3 cell that had been cultured in advance and become confluent in 75F flask (BD product of Falcon) was immersed in 0.05% mitomycin C solution for two hours so as to suppress the proliferation activity. Sequentially, they were was washed with a phosphate buffer solution (PBS) several times so as to remove mitomycin C, followed by treating with 0.05% trypsin-EDTA solution (PBS) so as to prepare a 3T3 suspension.

### 1-8. Cell culture and induction of mucosal epithelium

By using human amniotic membrane from which the epithelium had been scraped as a substrate, the oral mucosal epithelial cells and corneal epithelial cells were co-cultured with 3T3 cells that were subjected to the above-mentioned treatment by the following procedure. For culturing instruments, a 6-well culture dish (Coming, NY) and a culture insert (a container for inserting culture) (polycarbonate, average pore size: 3.0 µm, Coming NY) were used.
First of all, 3T3 cell suspension was seeded on the culture dish so that the cell density was about 1×10⁴ cells/cm² and cultured under conditions at 37°C and in 5%CO₂.
Furthermore, the amniotic membrane substrate was allowed to stand still to be attached on the culture insert with the side of the scraped epithelium upward, and dried for 10 minutes at room temperature. Thereafter, on the culture insert to which the amniotic membrane was attached, oral mucosal epithelial cell suspension and corneal epithelial cell suspension were seeded so that the cell density was about 1×10⁴ cells/cm².
After the above-mentioned operation, as shown in Fig. 1, the culture insert was disposed in the culture dish and 3T3 cells, oral mucosal epithelial cells and corneal epithelial cells were cultured in the same culture medium. Note here that Fig. 1 is a schematic cross-sectional view showing a state during culturing. In the culture dish 1, the culture insert 2 is placed and on the bottom surface of the culture dish 1, the 3T3 cell layer 5 is formed. Furthermore, on the bottom surface of the culture insert 2, the amniotic membrane 3 is placed, and the oral mucosal epithelial cells and corneal epithelial cells 4 are cultured thereon. Reference numeral 6 denotes a culture medium.
As the culture medium, a DMEM / Ham's F12 mixture medium (mixing volume ratio: 1:1) including 10% FBS, insulin (5 mg/ml), cholera toxin (0.1 nM), penicillin-streptomycin (50 IU/ml) and human recombinant epithelial cell growing factor (BGF) (10 ng/ml) was used.
The culture was carried out in the above-mentioned medium for seven days (Submerge). Thereafter, for inducing the mucosal epithelium, by a so-called Air-lifting method, culture was carried out for about three days. The Air-lifting method is a method of lifting the liquid surface of the culture medium to the surface of the oral mucosal epithelial cell layer formed on the amniotic membrane to bring the cell layer into contact with the air. During submerging, the culture medium was replaced with new one every other day and after carrying out the Air-lifting method, the culture medium was replaced with new one every day.
A multi-layered cell layer including 5 to 6 layers was formed by about 10 days culture(including three days of culture by the air-lifting method) according to the above-mentioned method.

### 1-9. Identification of cells constituting cell layer

Whether the cell layer on amniotic membrane, which has been obtained by the above-mentioned method, is formed as hybridization of the oral mucosal epithelial cells and the corneal epithelial cells was confirmed by the following procedure. Firstly, a Dil coloring agent was added to an oral mucosal epithelial cell suspension before seeding on the amniotic membrane and allowed to stand still for about 15 minutes at room temperature (Dil label). The thus obtained labeled oral mucosal epithelial cells together with the corneal epithelial cells were seeded on the amniotic membrane from which the epithelium had been removed. Thereafter, the culturing was carried out in the same conditions mentioned above. On day 1, day 3 and day 7 of culture, the formed cell layer was observed by using an optical microscope and a fluorescence microscope. The results are shown in Fig. 2 (Day 1), Fig. 3 (Day 3), and Fig. 4 (Day 7). In each figure, left image is an optical microscope image and right image is a fluorescence microscope image. On day 1, a state in which cells are excellently proliferated is shown (see left image of Fig. 2). Furthermore, a state in which Dil signals are scattered is observed (see right image of Fig. 2), showing that the oral mucosal epithelial cells and the corneal epithelial cells are present together. On day 3, a state in cells are arranged regularly (see left image of Fig. 3). Furthermore, a state in which Dil signals are scattered is still observed (see right image of Fig. 3), showing that two kinds of cells (the oral mucosal epithelial cells and the corneal epithelial cells) are proliferated in a form of hybridization so as to form a cell layer. On day 7, similar to the state on day 3, excellent cell proliferation and hybridization of cells are observed (see Fig. 4). Furthermore, in accordance with the cell proliferation and multi-layering, a region in which Dil signals are observed, is increased (see right image of Fig. 4).

### 1-10. Evaluation of histological properties of cell layer

The cell layer, which had been finally obtained by the above-mentioned methods (1-1 to 1-8), were observed by using an optical microscope. As a result, at the basal side (the side of the amniotic membrane) of the cell layer, a group of relatively cuboidal-shaped cells similar to the basal cell existed. Furthermore, it was confirmed that the cells of the outermost layer had a flat shape but included a nucleus and that the surface thereof was not cornified unlike the skin. As mentioned above, the optical microscope observation showed that the epithelium layer (corneal epithelial sheet) similar to the cornea was formed on the amniotic membrane.

Then, in order to examine the physiological property of the cell layer, immunostaining was carried out. After the obtained cell layer was cut into an appropriate size and frozen and embedded in an OCT compound. Then, the resultant compound was sliced with a cryostat to prepare slide sections. In immunostaining, the consideration on keratins, that is, respective cytoskeleton proteins were carried out. That is to say, keratin 1/10 specific to epidermis, keratin 3/12 specific to the cornea, and keratin 4/13 specific to mucosa were considered. The method will be described below. A slide section was washed with a phosphate buffer solution (PBS) and then blocking with 1% fetal bovine serum (FBS) was carried out to suppress the non-specific antibody reaction. Thereafter, an antibody against each keratin (primary antibody) was reacted at room temperature for one hour. After reaction, the slide section was washed with PBS containing triton-X for 15 minutes three times, followed by reacting with fluorescence labeling antibody (secondary antibody) at room temperature for one hour. After reaction, the slide section was washed with a phosphate buffer solution (PBS) for 15 minutes three times and sealed, followed by observing the tissue with a confocal microscope.
The antibody reactions of the respective keratins with respect to the cell layer will be described below. Firstly, for keratins 1 and 10 specific to epidermis, the staining was not observed (see left image of Fig. 5). On the other hand, the staining of keratin 3 specific to cornea was observed in a wide range (see middle image of Fig. 5). Keratin 3 was observed in the corneal epithelial cells and the oral mucosal epithelium and it was thought that the property thereof was maintained under the culture conditions. The staining of keratin 3 was strong in the upper part of the cell layer. The staining of keratin 12 was also observed in a wide range (see middle image of Fig. 5) and in particular, in the upper part of the cell layer, strong staining was observed. This staining property was caused by the corneal epithelial cell to be used, suggesting that the property thereof was maintained after culture.
For keratins 4 and 13 specific to mucosa, staining was observed in the almost entire region (see right image of Fig. 5).
From the above-mentioned results, as the histological property of the formed cell layer, in the aspect of the cytoskeletal, the keratin (keratins 3 and 12) specific to the cornea are maintained, showing the similarity to the corneal epithelium. Furthermore, unlike the epidermis, the cell layer has not been differentiated to cornification. It was confirmed that the cell layer had a feature of the not-cornified mucosal epithelium and simultaneously maintained the keratin specific to the cornea.

### 1-11. Transplantation experiment

In accordance with the above-mentioned methods (1-1 to 1-8), a sheet having a cell layer on the amniotic membrane (hereinafter, referred to as "corneal epithelial sheet") was produced. Specifically, firstly, oral mucosal epithelial cells (autologous cells) were prepared from a 6-week old Japanese white rabbit and corneal epithelial cells (allo corneal epithelial cells) were prepared from a different individual (a 6-week old Japanese white rabbit). Then, both cells were seeded on the amniotic membrane from which the epithelium had been removed, followed by culturing. Thus, a corneal epithelial sheet was obtained. Meanwhile, to the rabbit from which the oral mucosal epithelial cell had been harvested, all the conjunctival epithelium having a thickness of 100 µm were removed from 4-mm outside of the limbus by using a crescent knife. By this operation, since the epithelial cells containing corneal epithelial stem cells were lost, artificial exhaustion of the ocular surface stem cells was thought to be reappeared. Then, the above-mentioned corneal epithelium transplantation sheet was transplanted into the region slightly inner from the limbus. In transplantation, by using 10-0 nylon fiber was used to stitch the sheet to the peripheral tissue. After transplantation, on the graft, a therapeutic contact lens was placed. After the operation, antibiotics and steroid ophthalmic ointment were applied twice a day. At the time of transplantation, the ocular surface had a transparency the same as that of the corneal epithelium transplantation sheet before transplantation (the results are not shown in the drawings).

The ocular surface which had undergone the transplantation was observed on day 2, on the first week, and on the second week after transplantation. In addition, a fluorescein staining test was carried out. The fluorescein staining test was carried out by directly administering a test paper into which a moisture such as ophthalmic solution including an antimicrobial drug had been included and causing eyeblink twice or three times, followed by observing the fluorescein staining of the ocular surface. When the corneal epithelium remained, due to the tight intercellular adhesive structure, a fluorescein is not infiltrated and no staining by the fluorescein was observed.
On day 2 after the transplantation, the transplanted corneal epithelium transplantation sheet maintained transparency (see left image of Fig. 6). Furthermore, it was confirmed by fluoresceine staining that the corneal epithelial sheet remained on the ocular surface without being damaged (see right image of Fig. 6). Meanwhile, since the graft (corneal epithelial sheet) showed no staining of fluorescein, it was confirmed that the corneal epithelial sheet had a barrier function similar to the corneal epithelium. Furthermore, since by the fluoresceine staining, staining of fluoresceine was confirmed over the entire periphery of the graft, therefore it was confirmed that the tissue existing in the transplanted part was not contamination of the remaining conjunctival epithelium.
Note here that since cells of the corneal epithelium are tightly adhered to each other, the fluorescein staining agent does not invade from the surface and staining of fluorescein is not observed in fluorescein straining test. On the other hand, when the adhesion between cells becomes loosen or the barrier function is damaged by exfoliation of the cell itself, invasion of the fluorescein staining agent occur, and the tissues are stained. Therefore, by examining the staining property of fluorescein staining was examined, it can be confirmed whether or not the transplanted corneal epithelial sheet had the barrier function similar to the corneal epithelium.

On the other hand, one week after the transplantation, the graft (corneal epithelial sheet) also remained on the ocular-surface. Moreover, it was confirmed that the graft was expanded to the surrounding as compared with the state on day 2 after the transplantation (see left image of Fig. 7). Furthermore, it was confirmed that the graft showed no fluorescein staining and maintained a barrier function necessary for the corneal epithelium (see right image of Fig. 7). The transparency was also not changed from that observed on day 2 after the transplantation and was highly maintained (see left image of Fig. 7).
The condition of the ocular surface two weeks after the transplantation was not particularly changed from the condition after one week after the transplantation. That is to say, the graft (corneal epithelial sheet) remained on the ocular surface and the transparency thereof was high (see left image of Fig. 8). Furthermore, it was confirmed that the graft showed no staining of fluorescein (see right image of Fig. 8) and the barrier function was maintained.

From the above-mentioned results, it was demonstrated that the corneal epithelial sheet obtained by the method mentioned above had an excellent survival property and the survival property was maintained for a long time. Furthermore, it was confirmed that the corneal epithelial sheet extended to the surrounding after transplantation, exhibited a barrier function necessary to the corneal epithelium for a long time, and exhibited high transparency. That is to say, the corneal epithelial sheet obtained by the above-mentioned method excellently functioned as a substitute of the corneal epithelium and could be used as a transplant material for reconstructing the ocular surface in the case where the cornea was injured and damaged.

### 1-12. Evaluation of histological property of corneal epithelial sheet after transplantation

Next, the corneal epithelial sheet two weeks after the transplantation was extracted and the histological property thereof was examined. Fig. 9 shows a HE staining image of the corneal epithelial sheet (right image was expanded view). On the upper part of amniotic membrane (see marks *), similar to the corneal epithelium, a cell layer in which cells are regularly arranged is confirmed. In this cell layer, to the upper layer, the number of the flat-shaped cells is increased and the structure extremely similar to the corneal epithelium is maintained.
Fig. 10 shows the results of staining test with respect to various keratins. The staining property almost similar to that of the corneal epithelial sheet before transplantation was shown. That is to say, staining of keratins 1 and 10 specific to epidermis was not observed (see left image of Fig. 10), staining of keratins 3 and 12 specific to cornea was observed (see middle image of Fig. 10), and staining of keratins 4 and 13 specific to mucosa was also observed (see right image of Fig. 10). As mentioned above, it was confirmed that the corneal epithelial sheet maintained the keratins (keratins 3 and 12) specific to the corneal epithelium also after transplantation. These results strongly support that the corneal epithelial sheet will exhibit the same function as the corneal epithelium for a long time from the histological viewpoint.

### [Example 2]

### <Production of hybrid-type corneal epithelial sheet using human cells and evaluation thereof>

A human oral mucosal epithelial cell suspension was prepared by harvesting cells from a healthy volunteer after obtaining his/her agreement by the same method described in the above-mentioned 1-5 (harvest of oral mucosal epithelial cells). Meanwhile, a cornea obtained from an eye bank (Northwest eye bank) was treated by the same method described in the above-mentioned 1-6 (Harvest of corneal epithelial cells) so as to obtain a human corneal epithelial cell suspension. The thus prepared human oral mucosal epithelial cells and the corneal epithelial cells were co-cultured with 3T3 cells by using human amniotic membrane from which the epithelium had been scraped as a substrate. For about 14 days of culture (including three days of culture by the air-lifting method), a corneal epithelium-like cell layer was formed. Note here that the method of preparing the human amniotic membrane from which the epithelium had been scraped and co-culturing condition with 3T3 cells were the same as those described in the above-mentioned 1-1 (Harvest of amniotic membrane) to 1-4 (Treatment of amniotic epithelium).
When the form, structure and immunostaining, and the like, of the obtained cell layer were examined, the results were the same as those of a rabbit model. That is to say, the produced hybrid-type cultured cell layer had a multi-layered structure including 4 to 8 layers. No staining of keratins 1 and 10 specific to epidermis was observed, staining of keratins 3 and 12 specific to cornea was observed, and staining of keratins 4 and 13 specific to mucosa was also observed.
From the above-mentioned results, it was confirmed that in the case where human oral mucosal epithelial cells and human corneal epithelial cells were used, by hybridizing these cells, a corneal epithelium-like cell layer was formed.

### [Example 3]

### <Production of hybrid corneal epithelial sheet using amniotic membrane epithelial cells and evaluation thereof>

A human oral mucosal epithelial cell suspension was prepared by harvesting cells from a healthy volunteer after obtaining his/her agreement by the same method described in the above-mentioned 1-5 (harvest of oral mucosal epithelial cells). Meanwhile, a human amniotic membrane was obtained by the method described in the above-mentioned 1-1 (harvest of amniotic membrane) and 1-2 (treatment of amniotic membrane). Thereafter, the epithelial cells were separated by a treatment with 0.05% trypsin-EDTA solution (GIBCOBRL) for 15 minutes (amniotic membrane epithelial cell suspension). The thus prepared human oral mucosal epithelial cells and the human amniotic membrane epithelial cells were co-cultured with 3T3 cells by using human amniotic membrane from which the epithelium had been scraped as a substrate. For about 14 days of culture (including three days of culture by the air-lifting method), a corneal epithelium-like cell layer was formed. Note here that the method of preparing the human amniotic membrane from which the epithelium had been scraped and co-culturing condition with 3T3 cells were the same as those described in the above-mentioned 1-1 (Harvest of amniotic membrane) to 1-4 (Treatment of amniotic epithelium).
When the form, structure and immunostaining, and the like, of the obtained cell layer were examined, the produced hybrid-type cultured cell layer had a multi-layered structure including 4 to 8 layers. No staining of keratins 1 and 10 specific to epidermis was observed, staining of keratins 3 and 12 specific to cornea was observed, and staining of keratins 4 and 13 specific to mucosa was also observed.
From the above-mentioned results, it was confirmed that in the case where human oral mucosal epithelial cells and human amniotic membrane epithelial cells were used, when these cells were hybridized, a corneal epithelium-like cell layer was formed.

### Industrial Applicability

A corneal epithelial sheet of the present invention has a structure that is extremely similar to the structure of the corneal epithelium and shows an excellent survival property after transplantation. Furthermore, the corneal epithelial sheet of the present invention has a barrier function necessary for the corneal epithelium to exhibit its function and has high transparency. Thus, the corneal epithelial sheet provided by the present invention is extremely excellent as a transplantation material for re-constructing the corneal epithelium.
Recently, surgical reconstructive operations for refractory keratoconjunctive diseases have been progressed dramatically. With the appearance of amniotic membrane transplantation and cultured cornea transplantation, the operation technique has been almost established. However, since most of the refractory keratoconjunctive diseases are bilateral eye diseases, most transplantations use another individual's (allo) tissue, which requires a long-time and strict postoperative care including suppression of the rejection by administration of immunosuppressive drugs or prevention of bacterial infection. Under present circumstances, such things significantly deteriorate the quality of life (QOL) of patients. A transplantation using the hybrid-type corneal epithelial sheet of the present invention is an ocular-surface reconstructive operation using a tissue including an autologous (auto) cell. Therefore, the postoperative administration of immunosuppressive drugs is limited. In addition, since an autologous tissue is included, it is thought that the survival rate is more excellent as compared with a conventional allo transplantation, thus improving the QOL of a patient.

The present invention is not limited to the description of the above embodiments and Examples of the present invention. A variety of modifications, which are within the scopes of the claims and which can be easily achieved by a person skilled in the art, are included in the present invention.
All of the articles, publication of unexamined patent application, and Patent Gazette cited herein are hereby incorporated by reference.

## Claims

1. A corneal epithelial sheet comprising:
a cell layer including a first cell that is an autologous cell and a second cell whose origin is different from the first cell, the first cell and the second cell being layered.

2. The corneal epithelial sheet according to claim 1, wherein
the first cell is at least one autologous cell selected from the group consisting of a cell derived from oral mucosal epithelium, a cell derived from conjunctival epithelium, a cell derived from nasal mucosal epithelium, and a cell derived from other mucosal epithelium, as well as a cell derived from an undifferentiated cell capable of constructing any of the mucosal epitheliums; and
the second cell is at least one cell selected from the group consisting of a cell derived from corneal epithelium, a cell derived from conjunctival epithelium, and a cell derived from amniotic membrane epithelium.

3. The corneal epithelial sheet according to claim 2, wherein the first cell is a cell derived from oral mucosal epithelium.

4. The corneal epithelial sheet according to claim 2 or 3, wherein the second cell is a cell of another individual.

5. The corneal epithelial sheet according to any of claims 2 to 4, wherein the second cell is a cell derived from corneal epithelium or a cell derived from amniotic membrane epithelium.

6. The corneal epithelial sheet according to any of claims 1 to 5, further comprising a collagen layer, wherein the cell layer is formed on the collagen layer.

7. The corneal epithelial sheet according to claim 6, wherein the collagen layer is derived from amniotic membrane or amniotic membrane from which the epithelium has been removed.

8. The corneal epithelial sheet according to any of claims 1 to 7, wherein the cell layer comprises at least one of the following properties or characteristics;
cells of the outermost layer are not cornified;
cells of the outermost layer are flat-shaped; and
a barrier function is provided.

9. A process for producing a corneal epithelial sheet, comprising the following steps of:
a) separately preparing a first cell being an autologous cell and a second cell whose origin is different from that of the first cell;
b) seeding the first cell and the second cell on a collagen layer and culturing them; and
c) after proliferation of the first cell and second cell resulting in formation of a cell layer, bringing the surface of the cell layer into contact with the air.

10. The process according to claim 9, wherein
the first cell is at least one autologous cell selected from the group consisting of an oral mucosal epithelial cell, a conjunctival epithelial cell, a nasal mucosal epithelial cell, and other mucosal epithelial cell, as well as an undifferentiated cell capable of constructing any of the mucosal epitheliums; and
the second cell is at least one cell selected from the group consisting of a corneal epithelial cell, a conjunctival epithelial cell, and an amniotic membrane epithelial cell.

11. The process according to claim 10, wherein the first cell is an oral mucosal epithelial cell.

12. The process according to claim 10 or 11, wherein the second cell is a cell of another individual.

13. The process according to any of claims 10 to 12, wherein the second cell is a corneal epithelial cell or an amniotic membrane epithelial cell.

14. The process according to any of claims 9 to 13, wherein the step b is carried out under any of conditions,
1) in coexistence of supporting cells; and
2) in coexistence of supporting cells and in a state in which an isolation membrane with a pore size through which the supporting cell cannot pass exists between the supporting cells and the collagen layer.

15. The process according to any of claims 9 to 14, wherein the collagen layer is derived from amniotic membrane or amniotic membrane from which the epithelium has been removed.
